# EUROPÄISCHE PATENTANMELDUNG

(11) **EP 3 415 144 A1**
(43) Veröffentlichungstag der Anmeldung: **19.12.2018**
(21) Anmeldenummer: 18176838.3
(22) Anmeldetag: 08.06.2018
(51) Int. Cl.: A61K 31/198, A61K 31/375, A61K 31/385, A61K 31/519, A61K 31/7084, A61K 31/714, A61K 33/00, A61K 33/04, A61K 33/06, A61K 33/30, A61P 39/06, A61P 27/00

(54) **KOMBINATIONSTHERAPEUTIKUM ZUR BEHANDLUNG EINER MAKULADEGENERATION**

(30) Priorität: 13.06.2017 DE 102017005546
(71) Anmelder: Pries, Wolfgang, 86825 Bad Wörishofen (DE)
(72) Erfinder: Pries, Wolfgang, 86825 Bad Wörishofen (DE)
(74) Vertreter: Liebl, Thomas

(57) **Zusammenfassung**

Kombinationstherapeutikum zur Behandlung einer Makuladegeneration Es wird ein Kombinationstherapeutikum, umfassend Nicotinamid-Adenin-Dinukleotid-Hydrid (NADH) oder ein Salz davon und mindestens einen antioxidativen Zusatzstoff zur Verwendung bei der Behandlung einer Makuladegeneration beschrieben.

## Beschreibung

### Gebiet der Erfindung

Die Erfindung betrifft ein Kombinationstherapeutikum, umfassend Nicotinamid-Adenin-Dinukleotid-Hydrid (NADH) oder ein Salz davon und mindestens einen antioxidativen Zusatzstoff zur Verwendung bei der Behandlung einer Makuladegeneration.

### Hintergrund der Erfindung

Die Makula, auch "gelber Fleck" genannt, bezeichnet einen kleinen Bereich, der sich zentral auf der Netzhaut (Retina) des Auges befindet. Bei einem erwachsenen Menschen beträgt der Durchmesser der Makula in etwa 3 mm bis 5 mm. In ihrem Zentrum liegt die sogenannte Sehgrube, der Ort der höchsten Sehschärfe (Visus) mit der höchsten Dichte an farbempfindlichen Sinneszellen, den sogenannten Zapfen.

Die Makuladegeneration bezeichnet eine Gruppe von makulären Netzhauterkrankungen. Ein Funktionsverlust der unterschiedlichen, in der Makula befindlichen Zellen führt zum Nachlassen der zentralen Sehschärfe. Häufig sind beide Augen betroffen. Die Erkrankung kann jedoch an beiden Augen unterschiedlich verlaufen. Ebenso ist es möglich, dass nur ein Auge betroffen ist.

Die häufigste Form der Makuladegeneration ist die senile oder altersabhängige Makuladegeneration (AMD). Sie bezeichnet eine schmerzlose, fortschreitende Veränderung der Sehfähigkeit innerhalb des zentralen Gesichtsfeldes. Anfangs leiden Betroffene unter einem verschwommenen, sich allmählich vergrößernden, dunklen Fleck in der Mitte des Gesichtsfeldes. Außerdem nehmen das Kontrastempfinden, das Farbensehen sowie die Hell-Dunkel-Adaption ab. Die Blendempfindlichkeit ist erhöht und Konturen erscheinen verschwommen und verzerrt (Metamorphopsien).

Die altersabhängige Makula-Degeneration ist die Hauptursache für ein Erblinden bei Menschen im Alter von über 50 Jahren und verursacht ca. 32 % der Neuerblindungen, bei denen allerdings das periphere Gesichtsfeld erhalten bleibt. Durch die veränderte Altersstruktur der Bevölkerung mit einem steigenden Anteil von älteren Menschen hat sich die Zahl der Erkrankungen erheblich vergrößert. Nach Schätzungen sind weltweit 25 bis 30 Millionen Menschen an einer Makuladegeneration erkrankt. Jährlich kommen ca. 500.000 Neuerkrankungen hinzu. In Deutschland leiden schätzungsweise etwa 4 Millionen Menschen an einer Makuladegeneration. Die Sehverluste, die bis zum Erblinden fortschreiten können, schränken die selbstständige Lebensführung eines Patienten erheblich ein, so dass gesellschaftliche Probleme in Bezug auf Versicherungs- und Pflegeleistungen aufgrund der altersabhängigen Makuladegeneration absehbar sind.

Man unterscheidet zwei Formen der altersabhängigen Makuladegeneration: die häufiger vorkommende, langsam fortschreitende trockene Form und die aggressivere feuchte AMD. Die trockene AMD umfasst weiterhin ein Frühstadium und ein Spätstadium. Die Erkrankung wird durch Drusenbildung, d.h. kleine, gelbliche Ablagerungen unter der Netzhaut und Pigmentepithelveränderungen auf dem Augenhintergrund, gefördert. Drusen entstehen z.B. durch oxidativen Stress und schlechte Mikrozirkulation, gefolgt von Ablagerungen von extrazellulärem Material wie Immunkomplexen, Proteinen oder Lipiden unterhalb der Netzhaut. Kleine, harte Drusen treten bei über 98 % der Gesamtpopulation, auch bei gesunden Menschen, auf und können wieder abgebaut werden. Weiche Drusen hingegen werden meist erst im höheren Alter beobachtet. Am Entstehen von weichen Drusen ist ein vorgeschädigtes retinales Pigmentepithel beteiligt. Drusen können die irreversible Schädigung des Pigmentepithels noch weiter fördern. Eine Degeneration in Form von funktionellen und/oder morphologischen Veränderungen und eine Atrophie (Gewebeschwund) des Pigmentepithels führen zu einer Degeneration der oberhalb der Druse liegenden Zellen. Das Auftreten von weichen Drusen fördert meist den Beginn einer AMD. Drusen schränken die Sehschärfe nicht ein, sie verursachen jedoch Störungen der Farb- und Kontrastsensitivität. Im Anfangsstadium ist das Sehen nur geringfügig eingeschränkt und die Veränderungen schreiten nur langsam fort. Im Spätstadium gehen Sinneszellen und deren Ernährungszellen zu Grunde. Patienten mit fortgeschrittenem Stadium der trockenen AMD haben auch ein höheres Risiko die feuchte Form der AMD zu entwickeln.

Mit der aggressiveren feuchten AMD ist eine Sehschärfe-Verschlechterung verbunden. Bei einer geringen Anzahl von Patienten wurden auch Drusen festgestellt. Verursacht wird die feuchte AMD durch die Bildung neuer, abnormaler Blutgefäße, die als kleine Gefäßknospen unter der Netzhaut wachsen. Da diese Gefäße undicht sind, tritt Gefäßflüssigkeit in die Netzhaut aus und führt dort zum Anschwellen der Makula, was zu einem Makulaödem und Blutungen in dem instabil gebauten Gefäßsystem führt. Eine Narbenbildung bewirkt einen bleibenden Sehschärfenverlust.

Die Therapie orientiert sich an der Form der Makuladegeneration. Bei der feuchten AMD kommen eine photodynamische Therapie (PDT) und Gefäßwachstums-Inhibitoren (VEGF-Inhibitoren) zum Einsatz, die direkt in den Augapfel gespritzt werden und dort Gefäßneubildungen hemmen. Weiterhin können Gefäße mittels perkutaner Laserbestrahlung verschlossen und Blutschwämme durch netzhautchirurgische Eingriffe beseitigt werden. Diese Behandlungen sind aufwändig, verursachen hohe Behandlungskosten und müssen in individuellen Abständen wiederholt werden. In der Schulmedizin gibt es für die trockene AMD bislang keine Standardtherapie. Die verlorene Sehkraft kann nicht wieder hergestellt werden. Um einem Fortschreiten der Makuladegeneration vorzubeugen, wird Patienten eine vitaminreiche Ernährung empfohlen, die jedoch nicht ausreichend wirksam ist. Aus diesem Grund wird eine verbesserte und/oder effizientere Therapie zur Behandlung sowie Prophylaxe einer Makuladegeneration benötigt.

### Zusammenfassung der Erfindung

Die vorliegende Erfindung betrifft ein Kombinationstherapeutikum, umfassend Nicotinamid-Adenin-Dinukleotid-Hydrid (NADH) oder ein Salz davon und mindestens einen antioxidativen Zusatzstoff zur Verwendung bei der Behandlung einer Makuladegeneration.

Das Kombinationstherapeutikum zur Verwendung gemäß der Erfindung wird ferner für ein Infusionsintervall an aufeinanderfolgenden Tagen verabreicht, wobei NADH in oraler Form, unmittelbar gefolgt von dem mindestens einen antioxidativen Zusatzstoff in Form einer intravenösen Infusion über eine Dauer von ungefähr 1 bis 2 Stunden und unmittelbar gefolgt von NADH in oraler Form verabreicht wird.

### Detaillierte Beschreibung der Erfindung

In einem ersten Aspekt betrifft die Erfindung ein Kombinationstherapeutikum, umfassend Nicotinamid-Adenin-Dinukleotid-Hydrid (NADH) oder ein Salz davon und mindestens einen antioxidativen Zusatzstoff zur Verwendung bei der Behandlung einer Makuladegeneration.

Der Begriff "Kombinationstherapeutikum" wie hier verwendet bezeichnet ein Mittel, das zwei oder mehrere antioxidative Stoffe umfasst. Der Vorteil des Kombinationstherapeutikums ist eine verbesserte antioxidative Hauptwirkung, eine Erweiterung des Wirkungsspektrums durch organische und anorganische Antioxidantien, die sehr stark antioxidativ sowie intra- und extrazellular wirksam sind, und eine Verbesserung der pharmakokinetischen Parameter einer antioxidativen Therapie. Ein besonderer Vorteil ist, dass die verwendeten antioxidativen Stoffe eine synergistische Wirkung erzielen. Das Kombinationstherapeutikum erzielt eine höhere Wirksamkeit als bei Verwendung der einzelnen antioxidativen Stoffe. Die Therapie mit dem Kombinationstherapeutikum ist vergleichsweise preisgünstig und gut verträglich. Das Kombinationstherapeutikum ist ausschließlich aus Stoffen, die natürlicherweise im Körper des Menschen vorliegen, zusammengesetzt. Die antioxidativen Stoffe können gemeinsam in einer Zubereitung, in verschiedenen Zubereitungen gleicher und/oder unterschiedlicher Darreichungsformen vorliegen. Eine Zubereitung bezeichnet ein Produkt, das aus mindestens einem antioxidativen Zusatzstoff und Hilfsstoffen zusammengesetzt ist. Die flüssige Darreichungsform umfasst beispielsweise eine Lösung, eine Suspension oder eine Emulsion. Die feste Darreichungsform umfasst beispielsweise eine Kapsel oder eine Tablette. Vorteilhaft ist hierbei, dass der Wirkstoff gemäß seiner physikochemischen Eigenschaften in optimaler Darreichungsform vorliegt. Dies gewährleistet eine besonders gute Haltbarkeit und Lagerstabilität vor der Anwendung und vermeidet unerwünschte pharmakologische, physiologische, und/oder physikalisch-chemische Wirkungen untereinander oder mit dem Behältnis der Zubereitung. Dadurch werden nachteilige, wertmindernde und unerwünschte Wirkungen auf den antioxidativen Stoff vermieden. Der Begriff "Nicotinamid-Adenin-Dinukleotid-Hydrid" oder "NADH" wie hier verwendet bezeichnet ein Coenzym von wasserstoffübertragenden Enzymen. Es ist ein starkes, körpereigenes Antioxidationsmittel und überträgt ein Proton sowie zwei Elektronen. NADH schützt zelluläre und extrazelluläre Substanzen und Strukturen vor der schädigenden Wirkung von Sauerstoffradikalen im Körper. Es verbessert die Mikrozirkulation. NADH ist weiterhin an der zellulären Energiegewinnung beteiligt. Ferner stimuliert NADH dosisabhängig die Biosynthese von Interleukin-6, welches eine neuroprotektive Wirkung bei verschiedenen Schädigungen von Zellen, insbesondere Nervenzellen, aufweist.

Der Begriff "antioxidativer Zusatzstoff" wie hier verwendet bezeichnet einen Stoff, der eine antioxidative Wirkung zeigt und zusätzlich zum NADH verwendet wird. Ein antioxidativer Stoff verhindert oder verzögert bereits in geringer Konzentration die Oxidation von anderen Stoffen und Strukturen in der Zelle. Der antioxidative Zusatzstoff hat ein niedrigeres Redoxpotential als der zu schützende Stoff oder die zu schützende Struktur in der Zelle und wird daher vor dem zu schützenden Stoff oder der zu schützenden Struktur in der Zelle oxidiert. Der antioxidative Zusatzstoff wirkt zusammen mit NADH synergistisch. Der antioxidative Zusatzstoff unterstützt NADH bei der schützenden Wirkung auf zelluläre und extrazelluläre Stoffe und Strukturen vor der schädigenden Wirkung von Sauerstoffradikalen im Körper. Oxidativer Stress durch freie Sauerstoffradikale fördert die Makuladegeneration. Wenn der oxidative Stress verringert wird, ist es möglich das Fortschreiten der Makuladegeneration zu verlangsamen. Die unterschiedlichen Kombinationen der antioxidativen Zusatzstoffe zeigen einen geringeren antioxidativen Effekt im Vergleich zu Kombinationen der antioxidativen Zusatzstoffe mit NADH. Überraschenderweise zeigte jede Kombination von NADH mit antioxidativen Zusatzstoffen nicht nur eine prophylaktische Wirkung gegen den oxidativen Stress, sondern auch einen therapeutischen Effekt auf die Makuladegeneration. Die Symptome der Makuladegeneration, beispielsweise gemessen an der Sehschärfe, verbesserten sich bei der Anwendung einer Kombination von NADH mit antioxidativen Zusatzstoffen in Abhängigkeit der Behandlungsdauer um bis zu 20 %, bei manchen Patienten um bis zu 50 %. Nebenwirkungen wurden nicht beobachtet. Folglich ist eine Therapie mit dem erfindungsgemäßen Kombinationstherapeutikum vorteilhaft bei der Behandlung einer AMD. Weiterhin kann das Kombinationstherapeutikum das Risiko für einen Übergang von einer trockenen in eine feuchte AMD reduzieren. Das Kombinationstherapeutikum kann ebenso eine feuchte in eine trockene AMD zurückführen.

In einer bevorzugten Ausführungsform wird das Kombinationstherapeutikum zur Verwendung gemäß der Erfindung für ein Infusionsintervall an aufeinanderfolgenden Tagen verabreicht, wobei NADH in oraler Form, unmittelbar gefolgt von dem mindestens einen antioxidativen Zusatzstoff in Form einer intravenösen Infusion über eine Dauer von ungefähr 1 bis 2 Stunden und unmittelbar gefolgt von NADH in oraler Form verabreicht wird. NADH wird vorzugsweise als Kapsel zu Beginn und am Ende des Applikationsschemas verwendet.

Der Begriff "Infusionsintervall" wie hier verwendet bezeichnet eine bestimmte, individuell festzulegende Dauer, in der das genannte Applikationsschema angewendet wird. Das Infusionsintervall kann in individuell festzulegenden Abständen in gleicher oder verkürzter oder verlängerter Dauer wiederholt werden. Das Infusionsintervall wird vorzugsweise alle 8 Wochen wiederholt, weiter bevorzugt wird das Infusionsintervall alle 4 Wochen, weiter bevorzugt alle 3 Wochen, wiederholt. Die Infusionsdauer, während der die Infusionslösung in Abhängigkeit von der Infusionsgeschwindigkeit vollständig dem Patienten verabreicht wird, liegt zwischen 1 bis 2 Stunden, vorzugsweise bei 1 Stunde. Während der Infusionsdauer wird der Blutdruck des Patienten mehrfach kontrolliert. Vorteilhafterweise folgt eine einstündige Ruhephase nach dem Infusionsende. Durch die intravenöse Applikation wird eine Bioverfügbarkeit für die jeweiligen verwendeten antioxidativen Stoffe von 100 % erreicht. Die intravenöse Applikation ermöglicht höhere Dosierungen der antioxidativen Stoffe als z.B. bei peroraler Applikation.

In einer bevorzugten Ausführungsform wird das Kombinationstherapeutikum an 3 bis 8 aufeinanderfolgenden Tagen, vorzugsweise an 5 aufeinanderfolgenden Tagen verabreicht. Das Infusionsintervall orientiert sich am Stadium und an der Form der Makuladegeneration. Im fortgeschrittenen Stadium einer trockenen AMD ist ein längeres Infusionsintervall vorteilhaft. Am Ende jeden Infusionsintervalls ist es vorteilhaft die Sehschärfe zu bestimmen. Hierdurch ist eine speziell auf den Patienten angepasste Therapie möglich.

In einer bevorzugten Ausführungsform liegt NADH oder ein Salz davon in Form einer Kapsel, vorzugsweise einer magensaftresistenten Kapsel, vor. NADH ist ein säureinstabiler Feststoff. NADH wird bevorzugt als magensaftresistente Kapsel verwendet, um die Zersetzung des NADH in der Magensäure zu verhindern. Vorteilhaft ist, dass hierdurch NADH unzersetzt und daher zu einem größeren Anteil in den Körper aufgenommen wird.

Alternativ kann NADH auch kurz vor Applikationsbeginn zur Infusionslösung hinzugegeben werden. Weiterhin kann NADH auch in Form einer zweiten Infusionslösung verwendet werden, die separiert oder im kontinuierlichen Zufluss zur ersten Infusionslösung mit den antioxidativen Zusatzstoffen verabreicht wird.

In einer bevorzugten Ausführungsform entspricht der Gehalt von NADH oder eines Salzes davon 10 bis 1.000 mg NADH, vorzugsweise 10 bis 250 mg, weiter bevorzugt 10 bis 80 mg, am meisten bevorzugt 20 mg pro Dosiereinheit. Der Begriff "Dosiereinheit" wie hier verwendet bezeichnet eine verwendete Darreichungsform zur einmaligen Verwendung, z.B. 1 Kapsel oder 1 Infusionslösung. Die zu verabreichende Menge an NADH orientiert sich individuell an der Form und dem Stadium der Makuladegeneration ebenso an dem im Körper vorhandenen Antioxidantien-Status des Patienten, der sogenannten antioxidativen Kapazität. Die antioxidative Kapazität ist die Summe aller endogener und exogener Schutzmechanismen gegen den oxidativen Stress im Organismus. Vorzugsweise wird eine Gesamtmenge von etwa 20 mg bis 160 mg NADH, weiter bevorzugt 40 mg bis 80 mg NADH täglich verabreicht.

In einer bevorzugten Ausführungsform ist der mindestens eine antioxidative Zusatzstoff Ascorbinsäure, Glutathion, alpha-Liponsäure, Selen, Zink, Folsäure, Vitamin B12, Magnesium, Kalium und/oder L-Arginin, oder jeweils ein Derivat oder Salz davon. NADH wird mit mindestens einem antioxidativen Zusatzstoff kombiniert. Dies hat den Vorteil, dass die Kombination mit NADH einen synergistischen Effekt zeigt, der weit über die Effekte der Einzelsubstanzen hinausgeht. Durch das Verabreichen einer Kombination von verschiedenen antioxidativen Zusatzstoffen mit NADH wurden überraschend starke Verbesserungen der Sehschärfen der Patienten erzielt. Das Kombinationstherapeutikum kann neben NADH auch mehrere, insbesondere eine Vielzahl von antioxidativen Zusatzstoffen enthalten. Dabei werden die einzelnen antioxidativen Zusatzstoffe vorzugsweise in bestimmten Mengen und Verhältnissen zugesetzt. Weiter bevorzugt können die antioxidativen Zusatzstoffe in individuell festzulegenden Verhältnissen zugesetzt werden. Dies hat den Vorteil, dass die Dosierungen der Einzelstoffe im Kombinationstherapeutikum auf die Form und das Stadium der Makuladegeneration sowie den Antioxidantien-Status des Patienten zugeschnitten werden können.

In einer bevorzugten Ausführungsform ist der antioxidative Zusatzstoff Ascorbinsäure oder ein Salz davon und der Gehalt entspricht 100 bis 2.000 mg, vorzugsweise 600 bis 1.000 mg, weiter bevorzugt 800 bis 1.000 mg, am meisten bevorzugt 1.000 mg Ascorbinsäure pro Dosiereinheit. Die individuell zu verabreichende Menge an Ascorbinsäure orientiert sich an den bereits beschriebenen Parametern. Vorzugsweise wird eine Gesamtmenge von etwa 1.000 mg Ascorbinsäure je Behandlungstag verabreicht. Ascorbinsäure hat ein starkes Redoxpotential. Das Redoxpotential ist ein Maß für die Reduktions-/Oxidationsfähigkeit einer Substanz. Je negativer das Redoxpotential, desto stärker die Reduktionskraft. Ascorbinsäure wirkt als Cofaktor zahlreicher Enzymsysteme und ist ein wichtiger Bestandteil der Immunabwehr. Ascorbinsäure reichert sich bevorzugt in der Augenlinse an. Damit wirkt Ascorbinsäure nicht nur sehr gut im Blut antioxidativ, sondern auch im Auge.

In einer bevorzugten Ausführungsform ist der antioxidative Zusatzstoff Glutathion oder ein Salz davon und der Gehalt entspricht 100 bis 1.500 mg, vorzugsweise 100 bis 1.000 mg, weiter bevorzugt 500 bis 800 mg, am meisten bevorzugt 600 mg Glutathion pro Dosiereinheit. Die individuell zu verabreichende Menge an Glutathion orientiert sich an den bereits beschriebenen Parametern. Vorzugsweise wird eine Gesamtmenge von etwa 600 mg Glutathion je Behandlungstag verabreicht. Glutathion (GSH) ist ein Tripeptid, das aus den drei Aminosäuren Glutaminsäure, Cystein und Glycin besteht. Bei seiner antioxidativen Wirkung wird Glutathion oxidiert und geht von seiner monomeren Form GSH in ein Dimer GSSG über.

In einer bevorzugten Ausführungsform ist der antioxidative Zusatzstoff alpha-Liponsäure oder ein Salz davon und der Gehalt entspricht 100 bis 1.500 mg, vorzugsweise 100 bis 1.000 mg, weiter bevorzugt 500 bis 800 mg, am meisten bevorzugt 600 mg alpha-Liponsäure pro Dosiereinheit. Die individuell zu verabreichende Menge an alpha-Liponsäure orientiert sich an den bereits beschriebenen Parametern. Vorzugsweise wird eine Gesamtmenge von etwa 600 mg alpha-Liponsäure je Behandlungstag verabreicht. Die reduzierte Form der α-Liponsäure, die Dihydroliponsäure besitzt als Dithiol starke antioxidative Eigenschaften.

In einer bevorzugten Ausführungsform ist der antioxidative Zusatzstoff ein Selen-Salz und der Gehalt entspricht 50 µg bis 500 µg, vorzugsweise 100 µg bis 300 µg, weiter bevorzugt 200 µg bis 300 µg, am meisten bevorzugt 300 µg Selen pro Dosiereinheit. Die individuell zu verabreichende Menge an Selen orientiert sich an den bereits beschriebenen Parametern. Vorzugsweise wird eine Gesamtmenge von etwa 300 µg Selen je Behandlungstag verabreicht. Selen in Form von Selenocystein ist eine Aminosäure, die beispielsweise im aktiven Zentrum des Enzyms Glutathionperoxidase vorkommt. Die Glutathionperoxidase ist ein Bestandteil der zellulären Abwehr gegen oxidativen Stress. Außerdem ist Selen wegen der hohen Reaktivität mit Sauerstoff als Radikalfänger wichtig.

In einer bevorzugten Ausführungsform ist der antioxidative Zusatzstoff ein Zink-Salz und der Gehalt entspricht 1 mg bis 100 mg, vorzugsweise 5 mg bis 50 mg, weiter bevorzugt 10 mg bis 25 mg Zink pro Dosiereinheit. Die individuell zu verabreichende Menge an Zink orientiert sich an den bereits beschriebenen Parametern. Vorzugsweise wird eine Gesamtmenge von etwa 10 mg Zink je Behandlungstag verabreicht. Zink spielt eine wesentliche Rolle als strukturelle, katalytische oder regulatorische Komponente von Enzymen (z.B. der Dehydrogenasen), von Komplexbildnern und bei der zellulären und humoralen Immunantwort. Die höchsten Konzentrationen von Zink befinden sich im Augenhintergrund. Hierdurch ist Zink besonders gut geeignet, um im Auge antioxidativ zu wirken.

In einer bevorzugten Ausführungsform ist der antioxidative Zusatzstoff Folsäure oder ein Derivat oder ein Salz davon und der Gehalt entspricht 100 µg bis 10 mg, vorzugsweise 1 mg bis 10 mg, weiter bevorzugt 3 mg bis 6 mg, am meisten bevorzugt 5 mg Folsäure pro Dosiereinheit. Die individuell zu verabreichende Menge an Folsäure orientiert sich an den bereits beschriebenen Parametern. Vorzugsweise wird eine Gesamtmenge von etwa 5 mg Folsäure je Behandlungstag verabreicht. Folsäure spielt bei Wachstumsprozessen und der Zellteilung eine Rolle.

In einer bevorzugten Ausführungsform ist der antioxidative Zusatzstoff Vitamin B12 oder ein Derivat davon und der Gehalt entspricht 100 bis 1.500 mg, vorzugsweise 100 bis 1.000 mg, weiter bevorzugt 500 bis 1.000 mg, am meisten bevorzugt 1.000 mg Vitamin B12 pro Dosiereinheit. Die individuell zu verabreichende Menge an Vitamin B12 orientiert sich an den bereits beschriebenen Parametern. Vorzugsweise wird eine Gesamtmenge von etwa 1.000 mg Vitamin B12 je Behandlungstag verabreicht. Vitamin B12 spielt bei verschiedenen Stoffwechselvorgängen eine wichtige Rolle. Vitamin B12 ist beispielsweise beim Abbau bestimmter Fettsäuren beteiligt. Außerdem unterstützt Vitamin B12 die Blutbildung, indem es im Organismus gespeicherte Folsäure in seine aktive Form überführt.

In einer bevorzugten Ausführungsform ist der antioxidative Zusatzstoff ein Magnesium-Salz und der Gehalt entspricht 100 mg bis 1.000 mg, vorzugsweise 100 mg bis 500 mg, weiter bevorzugt 400 mg bis 500 mg Magnesium pro Dosiereinheit. Die individuell zu verabreichende Menge an Magnesium orientiert sich an den bereits beschriebenen Parametern. Vorzugsweise wird eine Gesamtmenge von etwa 500 mg Magnesium je Behandlungstag verabreicht. Magnesium wird von mehr als 300 Enzymen als Enzymbestandteil oder Coenzym benötigt und nimmt auf diesem Wege Einfluss auf die Zellregeneration, Sauerstoffnutzung und Energiegewinnung. Außerdem stabilisiert Magnesium biologische Membranen.

In einer bevorzugten Ausführungsform ist der antioxidative Zusatzstoff ein Kalium-Salz und der Gehalt entspricht 100 mg bis 1.000 mg, vorzugsweise 500 mg bis 1.000 mg, weiter bevorzugt 600 mg bis 800 mg, am meisten bevorzugt 780 mg Kalium pro Dosiereinheit. Die individuell zu verabreichende Menge an Kalium orientiert sich an den bereits beschriebenen Parametern. Vorzugsweise wird eine Gesamtmenge von etwa 780 mg Kalium je Behandlungstag verabreicht. Kalium ist an einer Vielzahl von physiologischen Vorgängen im Körper beteiligt, beispielsweise an der Regulation des Zellwachstums, der Beeinflussung von protektiven endothelialen Gefäßfunktionen, der Beeinflussung der Freisetzung von Hormonen, der Kohlenhydratverwertung und der Eiweißsynthese.

In einer bevorzugten Ausführungsform ist der antioxidative Zusatzstoff L-Arginin oder ein Salz davon und der Gehalt entspricht 100 mg bis 2.000 mg, vorzugsweise 300 mg bis 1.000 mg, weiter bevorzugt 300 mg bis 600 mg, am meisten bevorzugt 450 mg L-Arginin pro Dosiereinheit. Die individuell zu verabreichende Menge an L-Arginin orientiert sich an den bereits beschriebenen Parametern. Vorzugsweise wird eine Gesamtmenge von etwa 900 mg L-Arginin je Behandlungstag verabreicht. Der Körper kann nur aus Arginin das gefäßaktivierende Molekül Stickoxid (NO) bilden, welches die Weitung der Gefäße und so die Durchblutung und den Blutdruck steuert. Daher ist Arginin eine essentielle Aminosäure.

In einer bevorzugten Ausführungsform werden die antioxidativen Zusatzstoffe Zink und/oder Selen zeitlich versetzt verabreicht, wobei der Gehalt des Selen-Salzes 50 µg bis 500 µg, vorzugsweise 100 µg bis 300 µg, weiter bevorzugt 200 µg bis 300 µg, am meisten bevorzugt 300 µg Selen pro Dosiereinheit entspricht, und/oder der Gehalt des Zink-Salzes 1 mg bis 100 mg, vorzugsweise 5 mg bis 50 mg, weiter bevorzugt 10 mg bis 25 mg Zink pro Dosiereinheit entspricht. Der Begriff "zeitlich versetzt" wie hier verwendet bezeichnet das Verabreichen von Selen und Zink mit einem Abstand von drei bis vier Stunden.

In einer bevorzugten Ausführungsform werden die antioxidativen Zusatzstoffe Zink und/oder Selen oral verabreicht. Zwischen den Infusionsintervallen nehmen die Patienten Zink und/oder Selen in oraler Form, z.B. in Kapsel oder Tablettenform, ein. Dies hat den Vorteil, dass der Patient auch zwischen den Infusionsintervallen unabhängig von direkter ärztlicher Betreuung seinen Antioxidantien-Status konstant halten bzw. weiter aufbauen kann. Dies verbessert die Therapieerfolge.

In einer bevorzugten Ausführungsform werden die antioxidativen Zusatzstoffe Zink und/oder Selen vorzugsweise täglich und vorzugsweise für 8 Wochen, weiter bevorzugt für 4 Wochen, weiter bevorzugt für 3 Wochen nach einem Infusionsintervall verabreicht. Die Häufigkeit und Dauer der Verabreichung orientiert sich an der Form und dem Stadium der Makuladegeneration sowie am Antioxidantien-Status des Patienten.

In einer bevorzugten Ausführungsform sind die antioxidativen Zusatzstoffe Ascorbinsäure und Glutathion. Die Kombination von Ascorbinsäure und Glutathion mit NADH ist synergistisch wirksam im Vergleich zu den jeweiligen einzelnen Substanzen. Die individuell zu verabreichende Menge an Ascorbinsäure und Glutathion orientiert sich an den bereits beschriebenen Parametern. Vorzugsweise wird eine Gesamtmenge von etwa 1.000 mg Ascorbinsäure und eine Gesamtmenge von etwa 600 mg Glutathion je Behandlungstag verabreicht. Die Gabe von antioxidativen Zusatzstoffen mit unterschiedlichen antioxidativen Mechanismen potenziert die Wirksamkeit des Kombinationstherapeutikums.

In einer bevorzugten Ausführungsform sind die antioxidativen Zusatzstoffe Ascorbinsäure und Magnesium. Die Kombination von Ascorbinsäure und Magnesium ist synergistisch wirksam im Vergleich zu den jeweiligen einzelnen Substanzen. Vorteilhaft ist, wenn Ascorbinsäure und Magnesium gleichzeitig verabreicht werden. Damit wird ein optimaler, synergistischer Effekt von Ascorbinsäure und Magnesium erzielt. Die individuell zu verabreichende Menge an Ascorbinsäure und Magnesium orientiert sich an den bereits beschriebenen Parametern. Vorzugsweise wird eine Gesamtmenge von etwa 1.000 mg Ascorbinsäure und eine Gesamtmenge von etwa 500 mg Magnesium je Behandlungstag verabreicht. Magnesium und Ascorbinsäure bilden zusammen einen Komplex, der die Aufnahme des entstehenden Magnesiumascorbats in die Zellen verbessert.

In einer bevorzugten Ausführungsform werden die antioxidativen Zusatzstoffe Ascorbinsäure und Magnesium gleichzeitig verabreicht, wobei der Gehalt der Ascorbinsäure 100 bis 2.000 mg, vorzugsweise 600 bis 1.000 mg, weiter bevorzugt 800 bis 1.000 mg, am meisten bevorzugt 1.000 mg Ascorbinsäure pro Dosiereinheit entspricht, und wobei der Gehalt des Magnesiums 100 mg bis 1.000 mg, vorzugsweise 100 mg bis 500 mg, weiter bevorzugt 400 mg bis 500 mg Magnesium pro Dosiereinheit entspricht. Die gleichzeitige Gabe erhöht die Komplexbildung des Magnesiumascorbats und damit den Anteil des in die Zellen aufgenommenen Magnesiums und der Ascorbinsäure.

In einer bevorzugten Ausführungsform sind die antioxidativen Zusatzstoffe Ascorbinsäure, Glutathion, alpha-Liponsäure und Vitamin B12. Die Kombination dieser Zusatzstoffe ist synergistisch wirksam im Vergleich zu den jeweiligen einzelnen Substanzen. Die unterschiedlichen antioxidativen Mechanismen der antioxidativen Zusatzstoffe potenzieren die Wirksamkeit des Kombinationstherapeutikums.

NADH und die antioxidativen Zusatzstoffe werden in einer Basislösung gelöst bzw. verdünnt. Diese Basislösung ist vorteilhafterweise eine sterile isotonische (0,9 %) Kochsalzlösung in einer Menge von 50 ml bis 500 ml, vorzugsweise 50 ml bis 250 ml. Die isotonische Kochsalzlösung wird vorzugsweise bei Patienten mit Nieren- und/oder Herzinsuffizienz und bei dekompensierten Herzpatienten verwendet. Weiterhin ist die Basislösung vorteilhafterweise eine sterile, kolloidale Lösung von Hydroxyethylstärke (HES) sein. Vorzugsweise liegt HES in einer sterilen Elektrolytlösung vor. Der Gehalt von HES ist vorzugsweise 6 %. Die Elektrolyte umfassen Natrium, Kalium, Calcium, Magnesium, Chlorid, Acetat und L-Malat in physiologischen Konzentrationen. Die HES-Lösung wird vorzugsweise in einer Menge von 250 ml bis 500 ml verwendet.

### Beispiele

### Beispiel 1

### Herstellung der Infusionslösung und deren Anwendung zusammen mit NADH als Kombinationstherapeutikum

Die Infusionslösung wird aus kommerziell erhältlichen, flüssigen und sterilen Injektionslösungen der einzelnen antioxidativen Zusatzstoffe hergestellt.

### Beispiel 1a

Der Inhalt je einer Ampulle Ascorbinsäure (1.000 mg in 5 ml) und Glutathion (600 mg in 4 ml) werden zu 250 ml einer sterilen HES-Lösung gegeben.

### Beispiel 1b

Der Inhalt je einer Ampulle Ascorbinsäure (1.000 mg in 5 ml) und Magnesiumsulfat (entsprechend 493 mg Magnesium in 10 ml) werden zu 250 ml einer sterilen HES-Lösung gegeben.

### Beispiel 1c

Der Inhalt je einer Ampulle Ascorbinsäure (1.000 mg in 5 ml), Glutathion (600 mg in 4 ml), alpha-Liponsäure (600 mg in 24 ml) und Vitamin B12 (1000 µg in 2 ml) werden zu 250 ml einer sterilen HES-Lösung gegeben.

### Beispiel 1d

Der Inhalt je einer Ampulle Ascorbinsäure (1.000 mg in 5 ml), Glutathion (600 mg in 4 ml), alpha-Liponsäure (600 mg in 24 ml), Natriumselenit (entsprechend 300 µg Selen in 1 ml), Zink-D-Gluconat (entsprechend 10 mg Zink in 2 ml), Folsäure (5 mg in 2 ml), Vitamin B12 (1000 µg in 2 ml), Magnesiumsulfat (entsprechend 493 mg Magnesium in 10 ml) und Kaliumchlorid (entsprechend 780 mg Kalium in 20 ml) werden zu 250 ml einer sterilen HES-Lösung gegeben.

| **Bestandteile** | **Beispiel 1a** | **Beispiel 1b** | **Beispiel 1c** | **Beispiel 1d** |
|---|---|---|---|---|
| Ascorbinsäure | 1.000 mg | 1.000 mg | 1.000 mg | 1.000 mg |
| Glutathion | 600 mg | | 600 mg | 600 mg |
| Alpha-Liponsäure | | | 600 mg | 600 mg |
| Natriumselenit | | | | 300 µg |
| Zink-D-Gluconat | | | | 10 mg |
| Folsäure | | | | 5 mg |
| Vitamin B12 | | | 1.000 µg | 1.000 µg |
| Magnesiumsulfat | | 493 mg | | 493 mg |
| Kaliumchlorid | | | | 780 mg |
| HES-Lösung | 250 ml | 250 ml | 250 ml | 250 ml |

| | | | | |
|---|---|---|---|---|
| **Tabelle 1.** Zusammensetzung der Infusionslösungen der Beispiele 1a bis 1d. | | | | |

### Anwendung

Infusionslösungen dieser Zusammensetzung werden an fünf aufeinanderfolgenden Tagen für eine Infusionsdauer von 1 Stunde zusammen mit NADH und L-Arginin jeweils in Kapselform (je eine zu Beginn und zum Ende der Infusion) dem Patienten verabreicht. An das Ende der Infusion schließt sich eine Ruhephase des Patienten von 1 Stunde an.

### Beispiel 2

### Auswahl der Basislösung

Bei Patienten mit einer AMD handelt es sich meist um ältere, multimorbide Patienten. Daher erfolgen vor der Anwendung des Kombinationstherapeutikums eine klinische Untersuchung, die Bestimmung von verschiedenen Laborparametern und ein EKG. Hierdurch wird eine Niereninsuffizienz und/oder eine Herzinsuffizienz nachgewiesen, welche die Auswahl der Basislösung bestimmt. In Fällen einer Nieren- und/oder Herzinsuffizienz kommt der Einsatz einer volumenexpandierenden HES-Lösung nicht in Betracht, sondern es wird eine isotonische Kochsalzlösung als Basislösung verwendet, die keine volumenexpandierende Wirkung besitzt, um den Blutdruck und die Belastung des Herzkreislaufsystems nicht zu erhöhen.

Zeigt das EKG eine dauerhafte Herzfrequenz unter 55 Herzschläge pro Minute an, wird Magnesium in einer geringeren Dosierung als etwa 500 mg hinzugegeben, um die Herzfrequenz nicht weiter zu senken.

### Beispiel 3

### Augenärztliche Untersuchungen und Ergebnisse

Das Patientenkollektiv von 63 Patienten im Alter von 35 Jahren bis 87 Jahren wird in 3 Gruppen eingeteilt. Anhand der Gruppeneinteilung werden verschiedene Applikationsschema verwendet. Das Stadium wird aufgrund von Untersuchungen wie Sehschärfe (ETDRS-Buchstabentafeln, Landolt-Ringe), Verzerrungen (Amsler-Gitter), Kontrastsehen und Veränderungen an der Netzhaut (Funduskopie, Fluoreszenz-Angiographie, optische Kohärenztomographie) bestimmt. Die Gruppen umfassen Patienten mit trockener und feuchter AMD im Frühstadium sowie im fortgeschrittenen Stadium. Der Mindestvisus aller Patienten beträgt 0,4 im ETDRS-Äquivalent. Damit wird eine klinisch relevante Grundgesamtheit abgebildet. Die Studie wird prospektiv, placebo-kontrolliert und unter Doppelverblindung durchgeführt.

Gruppe 1 erhält das jeweilige Kombinationstherapeutikum für 7 Tage, Gruppe 2 erhält das jeweilige Kombinationstherapeutikum für 7 Tage plus die zusätzliche Gabe von Zink und Selen für 3 Wochen nach dem Infusionsintervall. Gruppe 3 erhält als Placebo die Basislösung ohne weitere Zusätze für 7 Tage. Die ärztliche Untersuchung wird immer an beiden Augen, vor Therapiebeginn, kurz vor Beginn des erneuten Infusionsintervalls sowie nach dem Ende des Infusionsintervalls durchgeführt. Die Studie wird für eine Dauer von 18 Monaten durchgeführt. Das Infusionsintervall wird in diesem Zeitraum im 4-Wochen-Rhythmus wiederholt.

**Tabelle 2. Verbesserung der Sehschärfe [%] der Probanden nach einer bestimmten Anzahl von Infusionsintervallen unter Verwendung des Kombinationstherapeutikums 1b.**

| Kombinationstherapeutikum 1b | Anzahl der Probanden | Verbesserung der Sehschärfe |
|---|---|---|
| 2 Infusionsintervalle ohne zusätzliche Gabe von Zink und Selen | 12 | 10-20 % |
| | 22 | 0 % |
| | 4 | Verschlechterung bis zu 3 % |
| 2 Infusionsintervall mit zusätzlicher Gabe von Zink und Selen | 5 | bis zu 28 % |
| 18 Infusionsintervalle mit zusätzlicher Gabe von Zink und Selen | 20 | bis zu 50 % |

Durch die Infusionsbehandlung mit dem Kombinationstherapeutikum der in Beispiel 1b genannten Zusammensetzung wird die Sehschärfe von 12 Probanden bereits nach zwei Infusionsintervallen um 10 bis 20 % verbessert. Auch die Anzahl und Dichte der Drusen sind verringert. Bei 5 Patienten mit AMD, die an das Infusionsintervall die orale Gabe von Zink und Selen für eine Dauer von 3 Wochen anschlossen, wird eine Verbesserung der Sehschärfe um bis zu 28 % nachgewiesen. Bei 22 Patienten wird eine Visus-Stabilisierung nachgewiesen. Bei 4 Patienten verschlechtert sich die Sehschärfe bis zu 3 % im Behandlungsverlauf. Bei 20 Patienten verbessert sich die Sehschärfe auf bis zu 50 % nach 18 Infusionsintervallen und einer oralen Gabe von Zink und Selen zwischen den Infusionsintervallen.

Damit wird gezeigt, dass das Kombinationstherapeutikum 1b eine Verlangsamung des Fortschreitens und auch einen Heilungsprozess der Makuladegeneration herbeiführt. Folglich reduziert das Kombinationstherapeutikum 1b das Risiko eines Übergangs von trockener zu feuchter AMD. Auch ein Zurückführen der feuchten AMD in die trockene AMD ist durch das Kombinationstherapeutikum möglich. Andere krankheitsbedingte Beeinträchtigungen und damit das subjektive Wohlbefinden der Patienten werden ebenso verbessert.

### Beispiel 4

### Bestimmung der totalen antioxidativen Kapazität des Blutes

Die totale antioxidative Kapazität wird vor Therapiebeginn und auch in regelmäßigen Abständen jeweils kurz vor Beginn des erneuten Infusionsintervalls sowie nach dem Ende des Infusionsintervalls gemessen.

Die intrazelluläre antioxidative Kapazität lässt sich hauptsächlich auf enzymatische Reaktionen zurückführen, wohingegen im extrazellulären Bereich hauptsächlich dem Körper zugeführte Antioxidantien diese Funktion wahrnehmen. Die antioxidative Kapazität des Plasmas kann als repräsentativ für das körpereigene Gleichgewicht von oxidierenden und antioxidativen Verbindungen angesehen werden. Die Laboruntersuchung wird mittels des TAS-Tests (Total Antioxidant Status Test; kommerziell erhältlich von Randox Laboratories) durchgeführt. Der Test gebraucht ABTS, 2,2'-Azinobis(3-ethylbenzothiazolin-6-sulfonat) und Wasserstoffperoxid, um ABTS-Radikalkationen in Gegenwart von Metmyoglobin (HX-FE(III)) als Peroxidase zu generieren. Antioxidantien in der Plasmaprobe vermindern die Bildung des Radikalkations mit blau-grüner Farbe. Das Ausmaß der entstehenden Färbung wird bei einer Wellenlänge von 600 nm absorptionsspektroskopisch bestimmt.

Die antioxidative Kapazität des Blutes liegt durchschnittlich bei dem größten Teil der Patienten zwischen 1,30 bis 1,77 mmol/l. Eine Korrelation der vorhandenen antioxidativen Kapazität mit einem Ansprechen auf das Kombinationstherapeutikum, messbar durch eine Verbesserung der Sehschärfe, wurde nicht beobachtet. Damit ist der gute Erfolg des antioxidativen Kombinationstherapeutikums unabhängig vom antioxidativen Status der Patienten. Die antioxidative Kapazität wird jedoch für die Dosierung des Kombinationstherapeutikums berücksichtigt, um eine zu hohe Dosierung der antioxidativen Stoffe zu vermeiden.

## Patentansprüche

1. Kombinationstherapeutikum, umfassend Nicotinamid-Adenin-Dinukleotid-Hydrid (NADH) oder ein Salz davon und mindestens einen antioxidativen Zusatzstoff zur Verwendung bei der Behandlung einer Makuladegeneration.

2. Kombinationstherapeutikum zur Verwendung nach Anspruch 1, wobei für ein Infusionsintervall an aufeinanderfolgenden Tagen
- NADH in oraler Form,
- unmittelbar gefolgt von dem mindestens einen antioxidativen Zusatzstoff in Form einer intravenösen Infusion über eine Dauer von ungefähr 1 bis 2 Stunden, und
- unmittelbar gefolgt von NADH in oraler Form
verabreicht wird.

3. Kombinationstherapeutikum zur Verwendung nach Anspruch 1 oder 2, wobei an 3 bis 8 aufeinanderfolgenden Tagen, vorzugsweise an 5 aufeinanderfolgenden Tagen verabreicht wird.

4. Kombinationstherapeutikum zur Verwendung nach einem der Ansprüche 1 bis 3, wobei NADH oder ein Salz davon in Form einer Kapsel, vorzugsweise einer magensaftresistenten Kapsel, vorliegt.

5. Kombinationstherapeutikum zur Verwendung nach einem der Ansprüche 1 bis 4, wobei der Gehalt von NADH oder eines Salzes davon 10 bis 1.000 mg NADH, vorzugsweise 10 bis 250 mg, weiter bevorzugt 10 bis 80 mg, am meisten bevorzugt 20 mg pro Dosiereinheit entspricht.

6. Kombinationstherapeutikum zur Verwendung nach einem der Ansprüche 1 bis 5, wobei der mindestens eine antioxidative Zusatzstoff Ascorbinsäure, Glutathion, alpha-Liponsäure, Selen, Zink, Folsäure, Vitamin B12, Magnesium, Kalium und/oder L-Arginin ist, oder jeweils ein Derivat oder Salz davon.

7. Kombinationstherapeutikum zur Verwendung nach einem der Ansprüche 1 bis 6, wobei der antioxidative Zusatzstoff Ascorbinsäure oder ein Salz davon ist und der Gehalt 100 bis 2.000 mg, vorzugsweise 600 bis 1.000 mg, weiter bevorzugt 800 bis 1.000 mg, am meisten bevorzugt 1.000 mg Ascorbinsäure pro Dosiereinheit entspricht.

8. Kombinationstherapeutikum zur Verwendung nach einem der Ansprüche 1 bis 7, wobei der antioxidative Zusatzstoff Glutathion oder ein Salz davon ist und der Gehalt 100 bis 1.500 mg, vorzugsweise 100 bis 1.000 mg, weiter bevorzugt 500 bis 800 mg, am meisten bevorzugt 600 mg Glutathion pro Dosiereinheit entspricht.

9. Kombinationstherapeutikum zur Verwendung nach einem der Ansprüche 1 bis 8, wobei der antioxidative Zusatzstoff alpha-Liponsäure oder ein Salz davon ist und der Gehalt 100 bis 1.500 mg, vorzugsweise 100 bis 1.000 mg, weiter bevorzugt 500 bis 800 mg, am meisten bevorzugt 600 mg alpha-Liponsäure pro Dosiereinheit entspricht.

10. Kombinationstherapeutikum zur Verwendung nach einem der Ansprüche 1 bis 9, wobei der antioxidative Zusatzstoff ein Selen-Salz ist und der Gehalt 50 µg bis 500 µg, vorzugsweise 100 µg bis 300 µg, weiter bevorzugt 200 µg bis 300 µg, am meisten bevorzugt 300 µg Selen pro Dosiereinheit entspricht.

11. Kombinationstherapeutikum zur Verwendung nach einem der Ansprüche 1 bis 10, wobei der antioxidative Zusatzstoff ein Zink-Salz und der Gehalt 1 mg bis 100 mg Zink, vorzugsweise 5 mg bis 50 mg, weiter bevorzugt 10 mg bis 25 mg pro Dosiereinheit entspricht.

12. Kombinationstherapeutikum zur Verwendung nach einem der Ansprüche 1 bis 11, wobei der antioxidative Zusatzstoff Folsäure oder ein Derivat oder ein Salz davon ist und der Gehalt 100 µg bis 10 mg, vorzugsweise 1 mg bis 10 mg, weiter bevorzugt 3 mg bis 6 mg, am meisten bevorzugt 5 mg Folsäure pro Dosiereinheit entspricht.

13. Kombinationstherapeutikum zur Verwendung nach einem der Ansprüche 1 bis 12, wobei der antioxidative Zusatzstoff Vitamin B12 oder ein Derivat davon ist und der Gehalt 100 bis 1.500 mg, vorzugsweise 100 bis 1.000 mg, weiter bevorzugt 500 bis 1.000 mg, am meisten bevorzugt 1.000 mg Vitamin B12 pro Dosiereinheit entspricht.

14. Kombinationstherapeutikum zur Verwendung nach einem der Ansprüche 1 bis 13, wobei der antioxidative Zusatzstoff ein Magnesium-Salz ist und der Gehalt 100 mg bis 1.000 mg, vorzugsweise 100 mg bis 500 mg, weiter bevorzugt 400 mg bis 500 mg Magnesium pro Dosiereinheit entspricht.

15. Kombinationstherapeutikum zur Verwendung nach einem der Ansprüche 1 bis 14, wobei der antioxidative Zusatzstoff ein Kalium-Salz ist und der Gehalt 100 mg bis 1.000 mg, vorzugsweise 500 mg bis 1.000 mg, weiter bevorzugt 600 mg bis 800 mg, am meisten bevorzugt 780 mg Kalium pro Dosiereinheit entspricht.

16. Kombinationstherapeutikum zur Verwendung nach einem der Ansprüche 1 bis 15, wobei der antioxidative Zusatzstoff L-Arginin oder ein Salz davon ist und der Gehalt 100 mg bis 2.000 mg, vorzugsweise 300 mg bis 1.000 mg, weiter bevorzugt 300 mg bis 600 mg, am meisten bevorzugt 450 mg L-Arginin pro Dosiereinheit entspricht.

17. Kombinationstherapeutikum zur Verwendung nach einem der Ansprüche 1 bis 16, wobei die antioxidativen Zusatzstoffe Zink und/oder Selen zeitlich versetzt verabreicht werden, wobei der Gehalt des Selen-Salzes 50 µg bis 500 µg, vorzugsweise 100 µg bis 300 µg, weiter bevorzugt 200 µg bis 300 µg, am meisten bevorzugt 300 µg Selen pro Dosiereinheit entspricht, und/oder der Gehalt des Zink-Salzes 1 mg bis 100 mg, vorzugsweise 5 mg bis 50 mg, weiter bevorzugt 10 mg bis 25 mg Zink pro Dosiereinheit entspricht.

18. Kombinationstherapeutikum zur Verwendung nach einem der Ansprüche 1 bis 17, wobei die antioxidativen Zusatzstoffe Zink und/oder Selen oral verabreicht werden.

19. Kombinationstherapeutikum zur Verwendung nach einem der Ansprüche 1 bis 18, wobei die antioxidativen Zusatzstoffe Zink und/oder Selen vorzugsweise täglich und/oder vorzugsweise für 8 Wochen, weiter bevorzugt für 4 Wochen, weiter bevorzugt für 3 Wochen, nach einem Infusionsintervall verabreicht werden.

20. Kombinationstherapeutikum zur Verwendung nach einem der Ansprüche 1 bis 19, wobei die antioxidativen Zusatzstoffe Ascorbinsäure und Glutathion sind.

21. Kombinationstherapeutikum zur Verwendung nach einem der Ansprüche 1 bis 19, wobei die antioxidativen Zusatzstoffe Ascorbinsäure und Magnesium sind.

22. Kombinationstherapeutikum zur Verwendung nach einem der Ansprüche 1 bis 19 und 21, wobei die antioxidativen Zusatzstoffe Ascorbinsäure und Magnesium gleichzeitig verabreicht werden und der Gehalt der Ascorbinsäure 100 bis 2.000 mg, vorzugsweise 600 bis 1.000 mg, weiter bevorzugt 800 bis 1.000 mg, am meisten bevorzugt 1.000 mg Ascorbinsäure pro Dosiereinheit entspricht und der Gehalt des Magnesiums 100 mg bis 1.000 mg, vorzugsweise 100 mg bis 500 mg, weiter bevorzugt 400 mg bis 500 mg Magnesium pro Dosiereinheit entspricht.

23. Kombinationstherapeutikum zur Verwendung nach einem der Ansprüche 1 bis 19, wobei die antioxidativen Zusatzstoffe Ascorbinsäure, Glutathion, alpha-Liponsäure und Vitamin B12 sind.
